# EUROPEAN PATENT SPECIFICATION

(11) **EP 1 709 199 B1**
(45) Date of publication and mention of the grant of the patent: **22.09.2010**
(21) Application number: 04820630.4
(22) Date of filing: 23.12.2004
(51) Int. Cl.: C12Q 1/68

(54) **DIRECT IDENTIFICATION AND MAPPING OF RNA TRANSCRIPTS**
DIREKTE IDENTIFIKATION UND MAPPING VON RNA-TRANSKRIPTEN
IDENTIFICATION ET MAPPAGE DIRECTS DE TRANSCRITS D'ARN

(30) Priority: 24.12.2003 US 532774 P
(43) Date of publication of application: 11.10.2006
(73) Proprietor: Advanomics Corp., Laval QC H7X 3K4 (CA)
(72) Inventor: SLILATY, Steve, N., (CA)
(74) Representative: MacLean, Martin Robert
(86) International application number: PCT/IB2004/004434
(87) International publication number: WO 2005/060344

(56) References cited:
- US-A- 6 074 824
- US-B1- 6 627 399
- GARDNER J F: "INITIATION PAUSING AND TERMINATION OF TRANSCRIPTION IN THE THREONINE OPERON REGULATORY REGION OF ESCHERICHIA-COLI" JOURNAL OF BIOLOGICAL CHEMISTRY, vol. 257, no. 7, 1982, pages 3896-3904, XP008083256 ISSN: 0021-9258
- SASAKI N. ET AL: 'Transcriptional sequencing: A method for DNA sequencing using RNA polymerase' PROC. NATL. ACAD. SCI. USA vol. 95, no. 7, 31 March 1998, pages 3455 - 3460, XP002390077
- KWON Y.S. ET AL: 'DNA sequencing and genotyping by transcriptional synthesis of chain-terminated RNA ladders and MALDI-TOF mass spectrometry' NUCLEIC ACIDS RES. vol. 29, no. 3, 01 February 2001, pages 1 - 6, XP008071121
- KLEMENT J.F. ET AL: 'Sequencing of DNA using T3 RNA polymerase and chain terminating ribonucleotide analogs' GENE ANAL. TECHN. vol. 3, no. 4, 1986, pages 59 - 66, XP000674971
- KUNDU T.K. ET AL: 'Promoter selectivity of Escherichia coli RNA polymerase sigmaF holoenzyme involved in transcription of flagellar and chemotaxis genes' J. BACTERIOL. vol. 179, no. 13, July 1997, pages 4264 - 4269, XP008071113
- CARDON L.R. ET AL: 'Comparative DNA sequence features in two long Escherichia coli contigs' NUCLEIC ACIDS RES. vol. 21, no. 16, 11 August 1993, pages 3875 - 3884, XP001536965

### SEQUENCE LISTING

<110> Slilaty, Stephen
<120> DIRECT IDENTIFICATION AND MAPPING OF RNA TRANSCRIPTS
<130> 41071.0PCT
<150> US 60/532,774
   <151> 2003-12-24
<160> 3
<210> 1
   <211> 389
   <212> DNA
   <213> artificial
<220>
   <223> pTS1 vector DNA
<400> 1
   gcttgcaagg  atcctaggcc  tgaagcttgt  cgacgaattc  acccgggaag  atcttgctta   60
   cgtacgcgtg  gtaccatgca  ttctccctat  agtgagtcgt  attatgcgcg  caattcactg  120
   gccgtcgttt  tacaacgtcg  tgactgggaa  aaccctggcg  ttacccaact  taatcgcctt  180
   gcagcacatc  cccctttcgc  cagctggcgt  aatagcgaag  aggcccgcac  cgatcgccct  240
   tcccaacagt  tgcgcagcct  gaatggcgaa  tggcgcctga  tgcggtattt  tctccttacg  300
   catctgtgcg  gtatttcaca  ccgcatatgg  tgcactctca  gtacaatctg  ctctgatgcc  360
   gcatagttaa  gccagccccg  acacccgcc 389
<210> 2
   <211> 540
   <212> DNA
   <213> artificial
<220>
   <223> pTS1 vector DNA
<400> 2
   tacgtaagca agatcttccc gggtgaattc gtcgacaagc ttcaggccta ggatccttgc 60
   gcaagctagc ctgcaggagc tctctccctt tagtgagggt taatttgcgc gcacagcttg 120
   gcgtaatcat ggtcatagct gtttcctgtg tgaaattgtt atccgctcac aattccacac 180
   aacatacgag ccggaagcat aaagtgtaaa gcctggggtg cctaatgagt gagctaactc 240
   acattaattg cgttgcgctc actgcccgct ttccagtcgg gaaacctgtc gtgccagctg 300
   cattaatgaa tcggccaacg cgcggggaga ggcggtttgc gtattgggcg ctcttccgct 360
   tcctcgctca ctgactcgct gcgctcggtc gttcggctgc ggcgagcggt atcagctcac 420
   tcaaaggcgg taatacggtt atccacagaa tcaggggata acgcaggaaa gaacatgtga 480
   gcaaaaggcc agcaaaaggc caggaaccgt aaaaaggccg cgttgctggc gtttttccat 540
<210> 3
   <211> 18
   <212> DNA
   <213> E. Coli B-lactamase gene
<220>
   <223>
<400> 3
   ggtgaaagtg atgctgaa 18

## Description

### FIELD OF THE INVENTION

The present invention relates generally to the field of transcriptome analysis and particularly to identification of RNA transcripts generated from DNA obtained from a population of cells.

### BACKGROUND OF THE INVENTION

The central dogma of molecular biology defined a general pathway where expression of genetic information stored in DNA is transcribed into transient mRNA and decoded on ribosomes with the help of adapter tRNA to produce proteins, which in turn perform all cell functions. According to this view, RNA plays an accessory role. In view of this, scientists have been collecting DNA sequence information from different organisms for decades. The highlight of this quest has undoubtedly been the joint international effort of the Human Genome Project to sequence, identify and determine the structure, regulation and function of the estimated 40,000 genes and their products. Therefore, once the human genome sequence was decoded, the field of proteomics, which is the study of all proteins within a cell, has recently gained enormous attention as the tool for advancing our understanding of the molecular basis of life and disease.

However, over 20 years ago, the discovery of the catalytic properties of the RNA subunit of ribonuclease P and the self-splicing activity of group I introns (Stark et al., 1978) suggested that the functions of RNA go beyond a passive role in the expression of protein-coding genes. In this regard, genomic analysis shows that when the complexity of an organism increases, the protein-coding contribution of its genome decreases (Szymanski & Barciszewski, 2002). It is estimated that 98 % of the transcriptional output of eukaryotic genomes is RNA that does not encode proteins (Mattick, 2001), including introns and transcripts from non-protein coding genes, with the latter accosting for 50-75% of all transcription in higher eukaryotes (Mattick & Gagen, 2001). Therefore, in recent years, the importance of these non coding RNAs (ncRNA) that do not encode proteins but have cellular functions on their own or in complex with proteins, has begun to be addressed.

To date, a functional classification of these ncRNA includes two general categories: housekeeping ncRNAs and regulatory ncRNAs. Examples of housekeeping ncRNAs include: transfer RNA (tRNA); ribosomal RNA (rRNA); small nuclear RNA (snRNA), including spliceosomal RNAs implicated in pre-mRNA splicing; small nucleolar (snoRNA), some involved in rRNA processing but most in rRNA modification (Elceiri, 1999). Some housekeeping genes are transcribed from their own promoter and terminator signals by RNA polymerase II (i.e. U3, U8, U13) or by RNA polymerase III (i.e. RNAse P and MRP RNAs), but the large majority are excised from introns of pre-mRNA. Almost all fall into 2 families: the "C/D box" which uses base complementarity to guide site-specific rRNA 2'-O-ribose methylations (Kiss-Laszlo et al., 1996; Nicoloso et al., 1996; Tycowski et al., 1996) and the "H/ACA" which guides rRNA pseudouridylations (Ganot et al., 1997; Ni et al., 1997). Catalytic function seems to be provided by enzymes associated to the snoRNAs and the specificity of the target base on the rRNA is provided by base complementarity to the snoRNA (Lafontaine & Tollervey, 1998; Wenstein & Steitz, 1999).

Additional examples of housekeeping ncRNA include RNAse P RNA, which is involved in maturation of 5' ends of pre-tRNA; telomerase RNA, implicated in telomeric DNA synthesis; 4.5S RNA in bacteria, and its eukaryotic counterpart, 7SL RNA, which are mediators of protein export; mRNA, involved in trans-translation; and RNAse MRP, which functions in mitochondrial RNA processing.
Regulatory ncRNAs are mainly synthesized by RNA polymerase II and are polyadenylated and spliced (Erdmann et al., 2001). They may be divided into 2 groups, the transcriptional regulators and the post-transcriptional regulators.

Transcriptional regulators are involved in chromatin remodeling associated with X-chromosome inactivation, dosage compensation in eukaryotes (i.e. roX, Xist/Tsix) and in regulation of expression of imprinted genes (i.e. HI9, IPW, LIT I).
Post-transcriptional regulators are implicated in repression or stimulation of translation of regulated mRNAs in eukaryotic and prokaryotic cells via antisense RNA-RNA interaction (i.e. DsrA, micF, lin-4, let-7, microRNAs, HFE, LjPLP-IV), modulation of protein function via RNA-protein interactions (i.e. 6S RNA, oxy S, SRA) and regulation of RNA and protein distribution (i.e. Xlsirt, hsr-ω). As the number of known regulatory ncRNA continued to increase, they have been grouped in families like small non-coding RNAs (sncRNA), micro RNAs (miRNA), (a putative translational repression regulatory gene family), small temporal RNAs (stRNA, i.e. lin-4 and let-7 in C. elegans) and small interfering RNAs (siRNA), (double-stranded RNA cleaved into 21-25 nt that degrades homologous mRNA and leads to a loss-of-function phenotype by antisense complementarity(Elbashir et al., 2001; Hunter, 2000; Carthew, 2001; Sharp, 2001; Vance & Vaucheret, 2001).

Gene identification is mainly based on 3 methods: cDNA cloning and expressed sequence tags (EST) sequencing of polyadenylated mRNAs (Liang et al., 2000; Ewing & Green, 2000), identification of conserved coding exons by comparative genome analysis (Roest Crouillis et al., 2000), and computational gene prediction (International Human Genome Sequencing Consortium, 2001; Venter et al., 2001). However, these methods work best for large, highly expressed, evolutionary conserved protein-coding genes, and thus certainly underestimate the number of other genes, including small mRNA, since they are discarded by size from the RNA fraction used for preparing ESTs or cDNA, and they do not work for ncRNA, which produce transcripts that function directly as structural, catalytic or regulatory RNAs (Eddy, 1999, 2001; Erdmann et al., 2001a, b). Additionally, many RNA identification techniques rely on polyadenylation, a feature many biologically important RNAs lack.

Early detection of ncRNAs was limited to biochemically abundant species and by chance. For example, some ncRNA are associated with ribonucleoproteins, and were discovered when these proteins were immunoprecipitated with specific antibodies, i.e. U1, U2, U4, U5 and U6 snRNA (Lemer & Steitz, 1981; Yu et al., 1999; Burge et al., 1999). Others were isolated biochemically, sometimes deliberately, like snoRNA from nucleoli (Elcieri, 1999), others by chance, as when fractions of ribonucelase P were found to contain essential RNAs (Stark et al., 1978) or when the signal recognition "protein" was in fact found to contain a 7S RNA and was subsequently called "signal recognition particle" (Lewin, 1982; Walter & Blobel, 1982). An example of serendipitous identification of a ncRNA gene by genetics is that of lin-4 regulatory RNA in C. elegans, identified in a screen for mutations that affect the timing and sequence of postembryonic development (Horvitz & Sulston, 1980). Subsequently, another ncRNA with similar function was discovered, the small let-7 RNA, also thought to be a post-transcriptional negative regulator (Reinhart et al., 2000).

Later on, more systematic approaches were pursued and a pioneer study found a few new RNA genes and ORFs in the yeast by doing Northern blots probed for expression transcripts in very large intergenic regions and by searching for consensus sequences of RNA polymerase III promoters (Olivas et al., 1997). Recent studies have carried out ncRNA gene identification screens along 3 main lines.

The first type of screen for ncRNA gene identification is with the use of specially designed cDNA cloning screens for very small RNAs. To identify miRNA, for example, Lau et al. (2001) produced and sequenced a C. elegans cDNA library enriched for tiny RNA with 5'-monophosphate and 3'-hydroxyl termini. Lee and Ambros (2001) used a size-selected C. elegans cDNA library in addition to a computational approach to look for conserved sequences that can be folded into a stem loop similar to the lin-4 and let-7 precursors and Lagos-Quintana et al. (2001) used size-selected cDNA libraries in humans and Drosophila.

The second type of screen for ncRNA gene identification is by the use of a general ncRNA gene-finding approach using computational comparative genomics. For example, Argaman et al. (2001) computationally analyzed intergenic regions in E. coli to identify loci that have promoter consensus sequences recognized by the major RNA polymerase σ70 factor within a 50-400 nucleotide (nt) distance of a terminator and which are significantly conserved in other bacterial genomes. In other approaches, Wassarman et al. (2001) looked for intergenic regions conserved in different genomes and their separation from the ORFs, the presence of putative promoter and terminator signals and significant RNA expression detected on whole genome high-density oligo-probes arrays, while Rivas et al. (2001) developed an algorithm to identify regions that conserve some particular type of RNA structure.

The third type of screen for ncRNA gene identification is by cDNA cloning and sequencing tailored to find new ncRNAs. In this approach, termed Experimental Rnomics, total RNA was isolated and size selected (i.e. 50-500) on denaturing PAGE, eluted and reverse transcribed for cDNA by tailing with CTP using polyA-polymerase to generate specialized cDNA libraries (Tang et al., 2002; Huttenhofer et al., 2001). Following amplification by PCR, they were spotted in high-density arrays of filters hybridized with radiolabeled oligonucleotides directed against the most abundant known ncRNA. The clones exhibiting the lowest hybridization scores were sequenced and those that did not map to any previously annotated genes were considered as potential ncRNA candidates. To analyze its expression, Northern blots were done using oligonucleotides complementary to the respective RNA sequence.

Although these methods have been useful in identifying new ncRNA, they also have important limitations. For example, computational identification of ncRNA is hampered by a lack of ORFs to aid in detection. Further, relying only on known conserved sequence/structural motifs will unlikely yield the complete set of ncRNA within a given organism. Searches limited to intergenic regions may be limited too because a number of hypothetical ORFs might not actually encode a protein but a ncRNA. In addition, ncRNA encoded by antisense transcripts located within known protein genes or ncRNA overlapping an ORF also would be missed when computer searches are limited to intergenic regions. Those based on the consensus sequence of major factors like σ70, will miss other factors like stress-specific transcription factors and/or alternative σ factors.

Even though experimental RNomics may be able to overcome some of the problems faced by computational analysis, this approach also contains limitations of its own, since screens only comprise RNAs ranging from 50-500 nt. However, sizes ofncRNA range from very large i.e. 17 kb for Xist (Brown et al., 1992) to extremely small (21-23 nt) (Ambros, 2001) and many would be missed by this method. In addition, the size of the ncRNAs determined by Northern blot analysis were in many instances larger compared to the sizes of the cDNA, either due to the strategy used to construct the libraries, which interferes with cloning at the 5'-ends of the novel ncRNAs or to the fact that RNA structure or modification impeded a complete conversion of the ncRNA into cDNA. Therefore, there is a need for a new method to identify RNAs missed in such conventional genetic screens, including ncRNA and small mRNA. Further, to provide a more realistic picture of RNA transcripts generated in vivo, there is need for developing a method which uses wild type RNA polymerases from the cell population/tissue in which RNA transcript generation is to be analyzed.

Sasaki et al. (Proc. Natl. Acad. Sci USA, Vol. 95, pp. 3455-3460, March 1998) describe a method for DNA sequencing using RNA polymerase.

Kwon et al. (Nucleic Acids Research, 2001, Vol. 29, No. 3 e11) describe DNA sequencing and genotyping by transcriptional synthesis of chain-terminated RNA ladders and MALDI-TOF mass spectrometry.

Gardner (J. Biol. Chem., Vol. 257, No. 7, pp. 3896-3904, 1982) describes initiation, pausing, and termination of transcription in the threonine operon regulatory region of *Escherichia coli.*

The invention provides a method for direct identification of RNA transcripts generated from a DNA sample obtained from a eukaryotic or prokaryotic cell population, wherein the DNA sample comprises a genomic library or a portion of a genomic library, comprising the steps of: a) preparing DNA fragments from the DNA sample such that each fragment has only one RNA polymerase promoter and wherein each RNA polymerase promoter is endogenous to the genome of the cell population; b) providing a wild type RNA polymerase, wherein the RNA polymerase is endogenous to the cell population; c) transcribing the DNA fragments with the wild type RNA polymerase in the presence of ribonucleoside triphosphates (rNTPs) and transcription terminating nucleotides to obtain transcription termination products; d) separating the transcription termination products; and e) determining the sequence of the RNA transcripts from the separated transcription termination products.

The invention also provides a method for direct identification of RNA transcripts generated from a DNA sample prepared from a eukaryotic or prokaryotic cell population, wherein the DNA sample comprises a genomic library or a portion of a genomic library, comprising the steps of: a) preparing DNA fragments from the DNA sample such that each fragment has only one RNA polymerase promoter and wherein each RNA polymerase promoter is endogenous to the genome of the cell population; b) transcribing the DNA fragments using a wild type RNA polymerase endogenous to the cell population to obtain RNA transcripts; and c) determining the sequence of the RNA transcripts.

The invention also provides a method for detecting differences in the sequence of RNA transcripts generated from DNA samples prepared from two different cell populations comprising the steps of: a) providing a first DNA sample from a first population of prokaryotic or eukaryotic cells and a second DNA sample from a second population of prokaryotic or eukaryotic cells; b) preparing DNA fragments from the first and second DNA samples to obtain a first and second set of DNA fragments such that each fragment of the first set of fragments has only one RNA polymerase promoter, wherein each RNA polymerase promoter is endogenous to the genome of the first cell population; and each fragment of the second set of fragments has only one RNA polymerase promoter, wherein each RNA polymerase promoter is endogenous to the genome of the second cell population; c) transcribing the first and second set of DNA fragments using a wild type RNA polymerase, to obtain a first and second set of RNA transcripts; d) determining the sequence of the first and second set of RNA transcripts; and e) comparing the sequence of the first and second set of RNA transcripts to detect differences between the first and second set of RNA transcripts.

This application describes a method for identifying RNA transcripts generated from DNA obtained from a cell population. The method comprises the steps of obtaining a DNA sample from a cell population, preparing segments of DNA such that each segment has only 1 promoter, allowing transcription to occur, and identifying and sequencing the resulting RNA transcripts. The DNA sample may be a genomic library or a portion of a genomic library.

Because each segment of DNA has an endogenous promoter, the identification of transcripts that are driven by the respective promoters provides identification of potential RNA transcripts that are expected to be generated in vivo, particularly if a wild type RNA polymerase also endogenous to the source of the cell population is used.

In one embodiment, the present method can be used identify, catalog and/or map RNA transcripts that can be generated in a cell population. This analysis can be carried out to obtain development related changes in RNA transcript population in an organism as well as changes due to onset of diseased conditions.

### BRIEF DESCRIPTION OF THE FIGURES

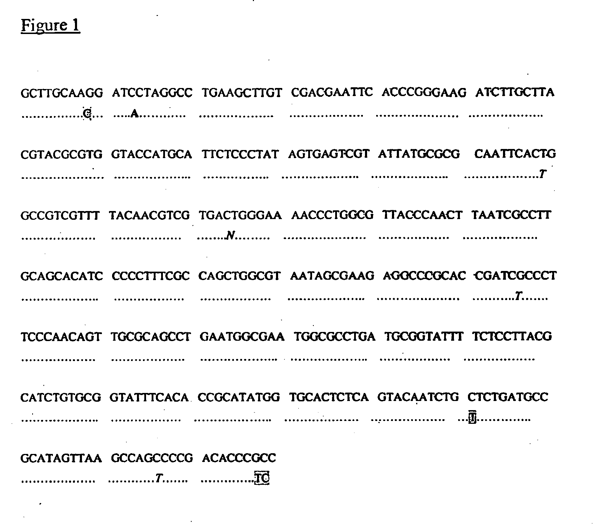

**Figure 1** is a sequence alignment of pTS1 DNA sequence transcribed under the control of T3 promoter compared with the expected sequence. The top line shows the predicted DNA sequence, while the bottom line displays the errors found in the experimental sequence, which are coded as follows: shaded nucleotides = mismatch; nucleotides in italics= insertion; nucleotides in bold= deletion.

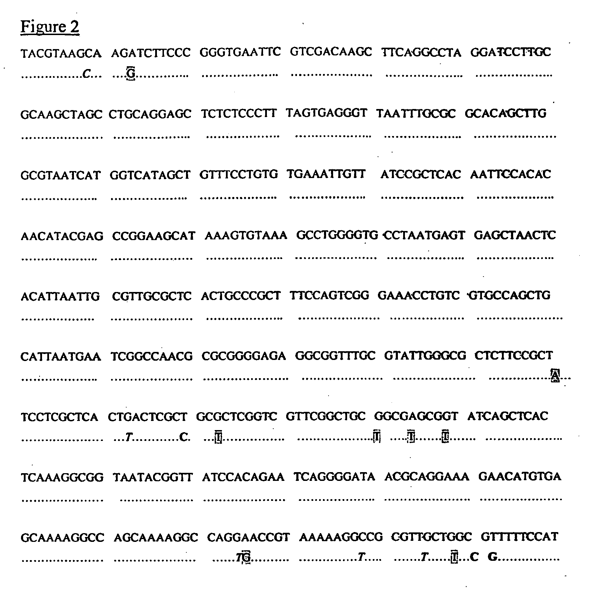

**Figure 2** is a sequence alignment of pTS1 DNA sequence transcribed under the control of T7 promoter. The top line shows the predicted DNA sequence, while the bottom line displays the errors found in the experimental sequence, wherein the nucleotides are coded as in the description of Figure 1.

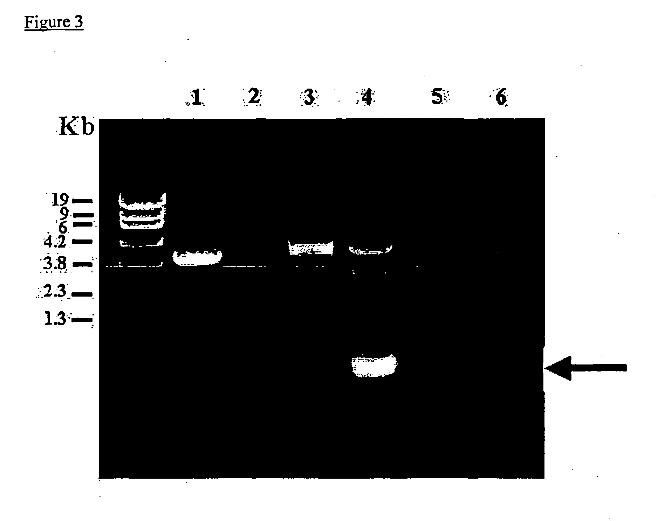

**Figure 3** is a photographic representation of electrophoretic separation of RNA transcription products. Linearized pUC 19 expressing β-galactosidase (lanes 1 and 2) or β-lactamase (lanes 3, 5 and 6) were incubated in the presence (lanes 2, 3 and 5) or the absence (lanes 1 and 6) of 4 units of E. coli RNA polymerase and the presence (lanes 2, 3, 5 and 6) or the absence (lane 1) of NTP/ terminator mixture for 5 hrs at 37°C. In lane 4, pTS1 DNA was incubated in the presence of T7 enzyme and NTP/terminator mixture. Following ethanol precipitation (lanes 4 and 5), reaction products were run on an agarose gel. The arrow marks the RNA transcription products.

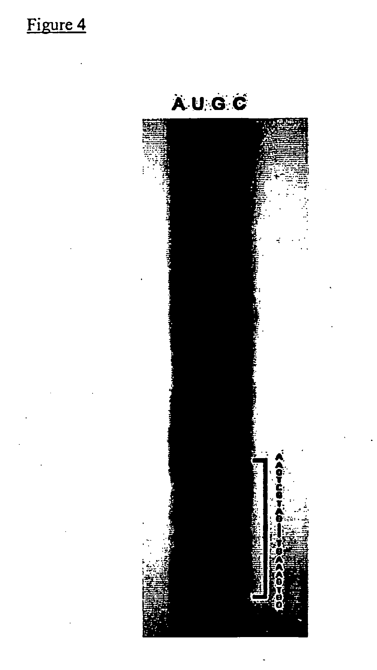

Figure 4 is a photographic representation of electrophoretic separation of specifically terminated RNA chains transcribed by *E. coli* RNA polymerase from the *β*-lactamase gene of pUC19. The sequence of RNA can be read in the 5' to 3' direction from the bottom of the gel according to the lanes labeled "A U G C". The sequence of the transcript is given vertically on the bottom right of the figure.

### DETAILED DESCRIPTION OF THE INVENTION

This invention provides a method for indirect transcriptome analysis in a cell population. Thus a method is provided for identifying RNA transcripts generated from DNA obtained from a cell population. DNA fragments are generated such that each fragment contains only one RNA polymerase promoter. Because each fragment of DNA has an endogenous promoter, the identification of transcripts that are driven by the promoter provides identification of possible RNA transcripts that are expected to be generated in vivo, particularly if a wild type RNA polymerase also endogenous to the source of the cell population is used.

In one embodiment, the method enables the determination of the sequence of RNA transcripts not normally detected by conventional RNA detection means, such as by cDNA generation. This determination is achieved because the method allows identification of all types of RNA transcripts that can be generated in vivo, rather than being limited to amplified transcripts with particular characteristics, such as polyadenylated tails typically required for generation of cDNA libraries. In another embodiment, the method of the invention enables the determination of the sequence of a complete set of possible RNAs transcribed in an organism, otherwise known as the transcriptome.

For the purposes of the specification and claims herein, a "wild type RNA polymerase" or "wild type polymerase" means an RNA polymerase that has not been genetically engineered, meaning the amino acid sequence of the polymerase has not been experimentally altered.

RNA polymerases used in the method of this invention may be any wild type RNA polymerases. Examples of suitable RNA polymerases are those found in prokaryotes such as E. coli, eukaryotic microbes such as Saccharomyces cerivisiae, and mammals. Further, the wild type polymerase may be provided in the form of a purified polymerase, a partially purified polymerase or in a transcriptionally active cell extract (TACE). Purified RNA polymerases are available commercially. For example, E. coli RNA polymerase holoenzyme can be purchased from Epicentre® (Madison, WI, USA). Some preparations of TACE are commercially available. For example, TACE prepared from rabbit reticulocytes can be purchased from Promega® (Madison, WI), Novagen® (Madison, WI) or Ambion® (Austin, TX). TACE prepared from human HeLa cells can be purchased from Promega® (Madison, WI). Desired TACE preparations not commercially available can be prepared using previously described methods (e.g. Manley et al., 1993; Sambrook et al., 2001; Blow, 1993; Veenstra et al., 1999).

DNA samples for use in the method of the invention can be obtained from any population of prokaryotic cells or any population of cells obtained from any organism and prepared by a variety of methods known to those skilled in the art. For example, DNA samples representing the entire genome or portions of the genome of an organism can be obtained by construction of shotgun libraries (Andersson et al., 1996), bacteriophage libraries, bacterial or yeast artificial chromosomes, and other suitable DNA vectors (See generally, Sambrook et al., 2001). Further, genomic libraries from various organisms are available commercially such as from Clontech®. In a preferred embodiment, the DNA sample is a genomic library or a portion of a genomic library divided into samples such that each sample comprises only one RNA polymerase promoter.

According to the method of the invention, once a complete or partial genomic DNA library or other suitable DNA sample, such as a polymerase chain reaction amplification product, is obtained it is subjected to transcription reactions or transcription sequencing reactions using a wild type RNA polymerase obtained from the same type of organism. For example, if the DNA sample was obtained from a population of cells from a eukaryote, a wild type eukaryotic RNA polymerase may be used so long as the polymerase is able to recognize and drive transcription from a promoter endogenous to the population of cells from which the DNA is obtained. In another embodiment, the wild type polymerase is obtained from an organism of the same species from which the DNA sample is obtained. For example, if the DNA sample is obtained from a population ofE. coli cells, the wild type RNA polymerase used would also be obtained from E. coli. In another embodiment, the wild type polymerase is obtained from the same population of cells from which the DNA sample is obtained. For example, if the DNA sample is obtained from a population of cells from an individual human, such as a tissue sample, the wild type polymerase is also obtained from a sample of that tissue.

Further, the method of the invention can be used to detect alterations in genomic DNA that result in the expression of different RNA transcripts between different populations of cells. For example, a wild type RNA polymerase obtained from an individual could be used to transcribe DNA from two different populations of cells (i.e., cancerous tissue compared to normal). The method disclosed herein would identify transcripts having different sequences when obtained from the cancerous, rather than normal cells due to alterations in the DNA that is transcribed.

Determination of the sequence of the RNA transcripts can be achieved in a variety of ways. In one embodiment, labeled RNA transcripts are produced using the method of the invention for use in hybridization assays. For example, commercially available radiolabeled ribonucleotides can be used in a transcription reaction to label the RNA transcript. A typical example of a radiolabeled ribonucleotide so used is α-³²P-UTP. Alternatively, the transcripts can be labeled with commercially available fluorescently labeled nucleotides, such as those available from Promega®, using methods known to those skilled in the art. The labeled transcripts can be hybridized to commercially available chips or DNA microarrays wherein the genome or a portion of the genome of the organism is represented on the chip of the array. Such DNA microarrays are available from, for example, Affymetrix, Inc. When hybridization between the labeled transcript and a location on the chip is detected, the sequence of the RNA can be determined by comparison with the sequence of the DNA at that location of the chip, wherein the RNA sequence is determined from the complementary with that DNA sequence. Similarly, the labeled transcripts can be used in hybridization assays to commercially available genomic library filters, wherein the location of hybridization is correlated with the sequence of DNA at the hybridization location and the sequence of the RNA transcript is determined from its complementarity with the DNA to which is has hybridized.

In another embodiment, a population of RNA transcripts produced from a DNA sample obtained from an organism can be identified using RNA sequencing reactions. Performing RNA sequencing on a DNA sample from a promoter endogenous to the organism from which the DNA sample is taken using a wild type polymerase is disclosed herein for the first time. Accordingly, this embodiment differs from that of U.S. Patent No. 6,627,399 which discloses the use ofpolyamines to accelerate RNA polymerase activity, but does not demonstrate RNA sequencing.

The synthesis of RNA transcription termination products for use in determining the sequence of an RNA transcript can be achieved by using ribonucleoside 5'-triphosphates (NTPs) such as adenosine triphosphate (ATP), guanosine triphosphate (GTP), cytosine triphosphate (CTP) and uracil triphosphate (UTP)) in combination with terminator nucleotides (3'-deoxynucleoside triphosphates (3'-dNTP's)) in a chain-terminating technique similar to that used in standard dideoxy DNA sequencing reactions.

The term "3'-dNTP" collectively refers to 3'-dATP, 3'-dGTP, 3'-dCTP and 3'-dUTP. The 3'-dNTPs are useful in the method of the invention because incorporation of a 3'-deoxyribose at the 3' end of an elongating transcription product results in termination of transcription. Termination occurs because the formation of a subsequent phosphodiester bond at the 3' position is precluded. As a result, a series of truncated chains are generated that are each terminated by the 3'-dNTP at each of the four possible nucleotide positions occupied by the corresponding base in the DNA sample. Separation of the terminated products according to their lengths (molecular weight) indicates the positions at which the base occurs in the DNA sample acting as the transcription template.

Once obtained, the transcription termination products are separated according to their relative molecular weights and the RNA transcript is identified by determining its sequence from the separated products. The separation of transcription termination products can be performed by any method which enables the separation of molecules having different molecular weights. Examples of such methods include gel electrophoresis and high pressure liquid chromatography.

Detection of the separated RNA transcription termination products can be facilitated by using labeled 5'-ribonucleoside NTPs. The label can be fluorescent or radioactive. Electrophorectically separated RNA transcription termination products having incorporated label can be visualized by a variety of methods, such as by exposure to film or analysis by phosphorimager when the label is radioactive. When the label is fluorescent, the termination products can be detected using automated polynucleotide sequencers, such as an ABI 377.

Once the sequence of the RNA is determined, the sequence can be used to locate the region of DNA that serves as the transcription template for the RNA transcript, as well as for RNA polymerase promoter sequences. For example, the sequence of the RNA transcript can be used to search through a database comprising the genomic sequence of the organism from which the DNA sample was obtained. Identification of a complementary location in the DNA determines the location of the coding region in the DNA which corresponds to the RNA transcript. Further, DNA sequences upstream of the transcriptional start site can be analyzed for homology with known promoter sequences or further analyzed to identify previously unknown promoters using standard molecular biology techniques.

The method of the present invention is demonstrated by the following Examples which are not meant to be limiting in any way.

### COMPARATIVE EXAMPLE 1

This Example demonstrates the identification of an RNA transcript using commercially available, genetically engineered bacteriophage polymerases. pTS1 transcription sequencing by T3 or T7 RNA polymerase (materials included with CUGA sequencing kit purchased from Nippon Genetech®, Toyama, Japan) was performed according to manufacturer's instructions. Briefly, 0.05 to 0.15 pmol of pTS1 DNA were incubated in a mixture containing MnCl₂, T3 or T7 enzyme solution, fluorescent-labeled nucleotide triphosphate (NTP)/terminator mixture and reaction buffer. The reaction was incubated at 37°C for 1 h, after which the labeled RNA was separated from the unbound material by adding 25 µl of 0.1M EDTA and 95% ethanol for 15 min at room temperature. Following centrifugation, the supernatant was discarded and the RNA-containing pellet, washed with 70% ethanol. After drying the pellet 15-25 µl of TSR buffer was added and the labeled RNA sequenced using an ABI PRISM® 310 Genetic Analyzer for which matrix standards were set-up according to manufacturer's instructions using the DT POP6 program from Dye terminator FS-kit. Briefly, each standard was reconstituted in 20 µl of TSR. A 47 x 50 cm capillary was used for sequencing on the ABI PRISM® 310 analyzer.

The sequencing results are presented in Figure 1 which shows the predicted and experimental DNA sequence of pTS1 (SEQ ID NO:1) transcribed by T3 polymerase. The top line of nucleotides shows the predicted DNA sequence. The bottom line indicates errors found in the experimentally obtained sequence, where shaded nucleotides represent mismatches, nucleotides in italics indicate insertions and nucleotides in bold designate deletions.

As can be seen, the experimental sequence is remarkably similar to the expected sequence, with approximately 3% errors, including the insertions, deletions or mismatches. The sequence was accurate up to approximately 450 nucleotides, after which the number of errors increased. A similar result was obtained when transcription controlled by T7 polymerase was tested and is provided in Figure 2 which has mismatches, insertions and deletions designated as in Figure 1. The experimental sequence for T7 sequencing (SEQ ID NO:2) was 96 % accurate with respect to the sequence predicted for a nucleotide chain length of about 580. In both cases, the majority of the errors were mismatch of C to T.

### EXAMPLE 2

This Example demonstrates that E. coli RNA polymerase can be used to transcribe an E. coli gene from an E. coli promoter in vitro in the presence and absence of terminating nucleotides.

*β*-lactamase or lacZ transcription controlled by E. coli RNA polymerase was performed as follows. Plasmid pUC19, which contains both the *β*-lactamase and the *β-*galactosidase (lacZ) genes, was linearized with either HindIII (for β-lactamase RNA expression) or SspI (for *β*-galactosidase RNA expression). Transcription was done essentially as described above in Example 1, with slight modifications as follows: 4 to 8 units of E. coli RNA polymerase were used; incubation times ranging from 1 to 16 hours were used; and 5 x E. coli RNA polymerase buffer containing 0.2 M Tris-HCl, 0.75 M KCl, 50 mM MgCl2 and 0.05 % Triton X-100 with or without 10 mM DTT was utilized.

The results of the transcription reactions are depicted in Figure 3. The figure is a photographic representation of an electrophoretic separation of transcription products through an agarose gel. The lanes show transcription and control reaction using linearized pUC19 digested for transcription of β-galactosidase (lanes 1 and 2) or β-lactamase (lanes 3, 5 and 6), which were incubated in the presence (lanes 2, 3 and 5) or the absence (lanes 1 and 6) of 4 units of E. coli RNA polymerase. These incubations were performed in either the presence (lanes 2, 3, 5 and 6) or the absence (lane 1) of NTP/ terminator mixture for 5 hs at 37°C. In lane 4, pTS1 DNA was incubated in the presence of T7 enzyme and NTP/terminator mixture. Following ethanol precipitation (lanes 4 and 5), reaction products were run on an agarose gel. The arrow marks the RNA transcription products.

As shown in this figure, transcription products can be detected for both β-lactamase and β-galactosidase on agarose gels, which is absent when no enzyme is included, thus demonstrating that E. coli polymerase can be used to transcribe a gene endogenous to E. coli under the control of an E. coli promoter.

### EXAMPLE 3

This Example demonstrates that the method of the invention can be used to identify an RNA transcript using a wild type polymerase to perform a transcription sequencing reaction on a DNA sample that contains an RNA promoter and gene endogenous to the organism from which the wild type polymerase is obtained.

pUC19 DNA (New England Biolabs) was linearized using *Hind*III (New England Biolabs) to inactivate the β-galactosidase promoter in order to drive RNA transcription only from β-lactamase promoter. E. coli RNA polymerase holoenzyme was purchased from Epicentre. This preparation is 100% saturated with sigma subunit (σ⁷⁰) and thus initiates RNA synthesis specifically at promoter sequences on native bacterial or bacteriophage DNA.

All four 3'-deoxyribonucleotide triphosphates were obtained from Trilink Biotechnology (San Diego, CA.). [α-³²P]UTP of specific activity 3000Ci/mmol was purchased from Perkin Elmer. Unlabeled ribonucleotide triphosphates were obtained from Epicentre.

Transcription reactions were carried out in 50µl reaction volume. A master mix for 5 reactions was prepared containing 50µl of E. coli RNA polymerase buffer (0.2M Tris-HCl, pH 8.0, 0.75M KCl, 50mM MgCl₂, 0.05% Triton); 5µg of template DNA, 10mM DDT, 4mM Spermidine (Sigma), 10µg/ml BSA (Sigma), 3mM MnCl₂ (Sigma), 400µM rNTPs (Epicentre). Volume was adjusted to 215µl with DEPC water. The solution was mixed gently by micropipette with out producing bubbles, then 100µCi of [α⁻³²P] UTP was added and mixed well. 45µl of this mix was dispensed into each of four Eppendorf tubes. For nucleotide sequence analysis, the reaction mixtures were then supplemented with 400µM 3'-dATP or 3'-dGTP or 3'-dCTP or 3'-dUTP, prior to the addition of 3 units of E. coli RNA polymerase. The reactions were incubated at 37°C in a dry incubator for 5 hours and terminated by adding 10µl of 200µM EDTA containing 1mg/ml Yeast tRNA (Sigma) as a carrier. Transcripts were precipitated from the reaction mixture by the addition of 200µl of ice-cold 4M acetic acid followed by incubation for 10 minutes at 0°C and centrifugation in an Eppendorf centrifuge for 15 minutes at 4°C. The pellets were washed with 500µl ice cold 2M acetic acid, centrifuged for 3 minutes, washed with 500µl of 70% ethanol, centrifuged for 3 minutes, and dried on the bench top. The RNA in each tube was resuspended in 6µl of Formamide Loading Dye containing (78% deionized formamide, 10mM EDTA pH 8, 0.1% Xylene Cyanol, 0.05% Bromophenol Blue in 1 X TBE buffer). The samples were stored at -20°C overnight.

Electrophoresis was performed in an International Biotechnologies Inc. sequencing apparatus (IBI STS-45i). The unit has a gel dimension of 43cm X 36cm and a 0.4mm thick gel was poured with a shark smooth comb. All solutions were made with DEPC treated water. The polyacrylamide gel was prepared using a ratio of acrylamide:bisacrylamide of 29:1 and was cast in 7M urea, 89mM Tris-borate, and 20mM EDTA (pH 8.0) and was run in 89mM Tris-borate and 20mM EDTA (pH 8.0). The upper and lower chambers of the electrophoresis apparatus contained approximately 530 ml of running buffer each. The buffer was not recirculated and the gel was not cooled. The gel was pre-run for 1 hour at 50 mA and the temperature was monitored by gel temperature indicator (Rose Scientific Ltd.). Immediately before loading the gel, samples were heated to 95°C in a block heater for 3 minutes then chilled on ice. Four micro liters of each sample were applied to the gel and electrophoresis was continued at 50mA with a temperature reaching up to 50°C and stopped when the bromophenol blue dye front was one inch from the bottom of the gel. After electrophoresis, the gel was fixed with 5% acetic acid (vol/vol) and 5% methanol (vol/vol), transferred to a 3MM Whatmann paper, covered with a Saran Wrap and exposed to a Kodak X-Omat film with a Corex Lighting-Plus intensifying screen from Du Pont at -80°C for 19hrs.

To verify product formation, a sample was run in agarose gel (results not shown) and detected by ethidium bromide. Transcription products were observed for E. coli RNA polymerase under standard transcription conditions (no chain terminators included). For the chain termination reactions using 3'deoxy-rNTPs, we determined that an effective ratio of rNTPs : 3'deoxy-rNTPs is 1:1 for all four rNTPs. At this ratio, termination was an infrequent event that generated sequencing ladders.

As shown in Figure 4, RNA transcript sequences obtained using E. coli RNA polymerase driving transcription from the E. Coli *β*-lactamase promoter on plasmid pUC19 were produced using these reaction conditions. The nucleotide sequence that was determined (SEQ ID NO:3) is in good agreement with the wild-type β-lactamase gene except for a gap of four nucleotides (Fig. 4).

Thus, this Example demonstrates that the method of the invention can be used to identify an RNA transcript using a wild type polymerase to perform a transcription sequencing reaction on a DNA sample that contains an E. coli promoter driving expression of an E. coli gene.

### REFERENCES

1. Altuvia S, Weisntein-Fischer D, Zhang A, Potow L, Storz G. A small, stable RNA induced by oxidative stress: role as a pleiotropic regulator and antimutator. Cell 90:45-53, 1997.
2. Ambros V. microRNAs: tiny regulators with great potential. Cell 107:823-826.
3. Andersson et al., Anal. Biochem. 236:107-13, 1996.
4. Argaman L, Hershberg R, Vogel J, Bejerano G, Wagner E, Margalit H, Altuvia S. Novel small RNA-encoding genes in the intergenic regions of Escherichia coli. CurrBiol 11:941-950, 2001.
5. Avner P, Heard E. X-chromosome inactivation: counting, choice and initiation. Nature Rev Gen 2:59-67, 2001.
6. Blow, J.J. Preventing Re-replication of DNA in a Single Cell Cycle: Evidence for a replication Licensing Factor. J. Cell. Biol. 122:993-1002, 1993.
7. Brown C, Hendrich B, Rupert J, Lafrenière R, Xing Y, Lawrence J, Willard H. The human XIST gene: analysis of a 17 kb inactive X-specific RNA that contains conserved repeats and is highly localized within the nucleus. Cell 71:527-542.
8. Burge C, Tuschi T, Sharp P. In: The RNA world, 2nd edition (eds. Gesteland R, Cech T, Atkins J), pp.525-560, Cold Spring Harbor Laboratory Press, New York, 1999.
9. Carter R, Dubchak I, Holbrook S. A computational approach to identify genes for functional RNAs in genomic sequences. Nuc Acids Res 29:3928-3938, 2001.
10. Carthew R. Gene silencing by double-stranded RNA. Curr Opin Cell Biol 13:244-248,2001.
11. Cavaillé J, Buiting K, Kiefmann M, Lalande M, Brannan C, Horsthemke B, Bachellerie J-P, Brosius J, Huttenhofer A. Identification of brain-specific and imprinted small nucleolar RNA genes exhibiting unusual genomic organization. Proc. Natl Acad Sci USA 97:14311, 2000.
12. Chamberlin, M.J., Kingston, R., et al. Isolation of Bacterial and Bacteriophage RNA Polymerases and Use in Synthesis of RNA in Vitro. Methods in Enzymology 101:540-568,1983.
13. Chen S, Lesnik E, Hall T, Sampath R, Griffey R, Ecker D, Blyn L. A bioinformatics based approach to discover small RNA genes in the Escherichia coli genome. Biosystems 65:157-177, 2002.
14. Eddy S. Noncoding RNA genes. Curr Opin Genet Devel, 9:695-699, 1999. - Eddy S. Non-coding RNA genes and the modem RNA world. Nature Rev 2:919929, 2001.
15. Elcieri G. Small nucleolar RNAs. Cell Molec Life Sci 56:22-31, 1999.
16. Erdmann V, Barciszewska M, Hochberg A, de Groot N, Barciszewski J. Regulatory RNAs. Cell Molec Life Sci 58:960-977, 2001.
17. Ewing B, Green P. Analysis of expressed sequence tags indicates 35,000 human genes. Nature Gen 25: 232-234, 2000.
18. Ganot P, Bortolin M, Kiss T. Site-specific pseudouridine formation in preribosomal RNA is guided by small nucleolar RNAs. Cell 89:799-809, 1997.
19. Horvitz H, Sulston J. Isolation and genetic characterization of cell-lineage mutants of the nematode Caenorhabditis elegans. Genetics 96:435-454, 1980.
20. Hsu, L.M., Vo, N.V., Kane, C.M. & Chamberlin, M.J. In Vitro Studies of Transcript Initiation by Escherichia coli. RNA Polymerase I. RNA Chain Initiation, Abortive Initiation, and Promoter Escape at Three Bacteriophage Promoters. Biochemistry, 42:3777-3786, 2003.
21. Hunter C. Gene silencing: shrinking the black box of RNAi. Curr Biol 10:R137-R140, 2000.
22. Hüttenhofer A, Kiefmann M, Meier-Ewert S, O"Brien J, Lehrach H, Bachellerie J-P, BrosiusJ. Rnomics: an experimental approach that identifies 201 candidates for novel, small, non-messenger RNAs in mouse. EMBO J, 20:2943-2953, 2001.
23. Hüttenhofer A, Brosius J, Bachellerie J-P. Rnomics: identification and function of small, non-messenger RNAs. Curr Opin Chem Biol 6:835-843, 2002.
24. International Human Genome Sequencing Consortium. Initial sequencing and analysis of the human genome. Nature 409:860-921, 2001.
25. Kiss-Laszlo Z, Henry Y, Bachellerie J-P, Calzergues-Ferrer M, Kiss T. Smite-specific ribose methylation of preribosomal RNA: a novel function for small nucleolar RNAs. Cell 85:1077-1088, 1996.
26. Klein R, Misulovin Z, Eddy S. Noncoding RNA genes identified in AT-rich hyperthermophiles. Proc Natl Acad Sci USA, 99:7542-7547, 2002.
27. Kobayashi S, Anzai K. An E-box sequence acts as a transcriptional activator for BC-1 RNA expression by RNA polymerase III in the brain. Biochem Biophys Res Commun 245:59-63, 1998.
28. Kramer, F.R. & Mills, D.R. RNA Sequencing with Radioactive Chain-Terminating Ribonucleotides. Proc. Natl. Acad. Sci. USA, 75:5335-5338, 1978.
29. Lafontaine D, Tollervey D. Birth of the snoRNPs : the evolution of the modification guide snoRNAs. Trends Biochem Sci 23:383-388, 1998.
30. Lagos-Quintana M, Rauhut R, Lendeckel W, Tuschi T. Identification of novel genes coding for small expressed RNAs. Science 294:853-858, 2001.
31. Lau N, Lim L, Weinstein E, Bartel D. An abundant class of tiny RNAs with probable regulatory roles in Caerorhabditis elegans. Science 294:858-862, 2001.
32. Lee R, Ambros V. An extensive class of small RNAs in Caenorhabditis elegans. Science 294:862-864, 2001.
33. Lerner M, Steitz J. Snurps and scyrps. Cell 25:298-300, 1981.
34. Levy M, Ellington A. RNA world: catalysis abets binding, but not vice versa. Curr Biol 11:R665-667, 2001.
35. Lewin R. Surprising discovery with a small RNA. Science 218:777-778, 1982.
36. Liang F, Holt I, Pertea G, Karamycheva S, Salzberg S, Quakenbush J. Gene index analysis of the human genome estimates approximately 120,000 genes. Nature Gen 25:234-240, 2000.
37. Manley, J.L. et al. In Vitro Transcription: Whole-Cell Extract. Methods Enzymol. 101:568-582, 1993.
38. Marker C, Zemann A Terhörst T, Kiefmann M, Kastenmayer J, Green P. Bachellerie J-P, Brosius J, Hüttenhofer A. Experimental Rnomics: identification of 140 candidates for small, non-messenger RNAs in the plant Arabidopsis thaliana. Curr Biol, 12:2002-2013, 2002
39. Mattick J. Non-coding RNAs: the architects of eukaryotic complexity. EMBO 2:986-991, 2001.
40. Mattick J, Gagen M. The evolution of controlled multitasked gene networks: the role of introns and other non-coding RNAs in the development of complex organisms. Mol Biol Evol 18:1611-1630, 2001.
41. Neff, N.F. & Chamberlin, M.J. Termination of Transcription by Escherichia coli Ribonucleic Acid Polymerase in Vitro. Effect of Altered Reaction Conditions and Mutations in the Enzyme Protein on Termination with T7 and T3 Deoxyribonucleic Acid. Biochemistry, 19:3995-3015, 1980.
42. Ni J, Tien A, Fournier M. Small nucleolar RNAs direct site-specific synthesis of pseudouridine in ribosomal RNA. Cell 89, 565-573, 1997.
43. Nicoloso M, Qu L, Michot B, Bachellerie J-P. Intron-encoded antisense small nucleolar RNAs: the characterization of nine novel species points to their direct role as guides for the 2'-O-ribose methylation of rRNAs. J Mol Biol 260:178-195, 1996.
44. Nierman, W.C. & Chamberlin, M.J. The Effect of Low Substrate Concentrations on the Extent of Productive RNA Chain Initiation from T7 Promoters A1 and A2 by Escherichia coli RNA Polymerase. JBC, 255:4495-4500,1980.
45. Olivas W, Muhlard D, Parker R. Analysis of the yeast genome: identification of new non-coding and small ORF-containing RNAs. Nucleic Acid Res. 25:4619-4625, 1997.
46. Parvin, J.D., Smith, F.I. & Palese, P. Laboratory Methods, Rapid RNA Sequencing Using Double-Stranded Template DNA, SP6 Polymerase and 3'-Deoxynucleotide Triphosphates. DNA, 5:167-171, 1986.
47. Pesole G, Liuni S, Grillo G, Saccone C. Structural and compositional features of untranslated regions of eukaryotic mRNAs. Gene 205:95-102, 1997.
   - Pesole G, Mignone F, Gissi C, Grillo G, Licciuli F, Liuni S. Structural and functional features of eukaryotic mRNA untranslated regions. Gene 276:73-81, 2001.
48. Raynolds, R., Bermudez-Cruz, R.M. & Chamberlin, M.J. Parameters Affecting Transcription Termination by Escherichia coli RNA Polymerase. J. Mol. Biol., 224:31-51, 1992.
49. Reinhart B, Slack F, Basson M, Pasquinelli A, Bettinger J, Rougvie A, Horvitz H, Ruvkun G. The 21-nucleotide let-7 RNA regulates developmental timing in Caenorhabditis elegans. Nature 403:901-906, 2000.
50. Rivas E, Eddy S. Noncoding RNA gene detection using comparative sequence analysis. BMC Bioinform 2:8, 2001.
51. Rivas E, Klein R, Jones T, Eddy S. Computational identification of noncoding RNAs in E. coli by comparative genomics. Curr Biol 11:1369-1373, 2001.
52. Roest Crollius H, Jaillon O, Bemot A, Dasilva C, Bourneau L, Fischer C, Fizames C, Wincker P, Brottier P, Quertier F, Saurin W, Weissenbach J. Estimate of human gene number provided by genomewide analysis using Tetraodon nigroviridis DNA sequence. Nature Gen 25:235-238, 2000.
53. Sambrook et al., Molecular Cloning: A Laboratory Manual. Cold Spring Harbor Laboratory, 3rd ed., 2001.
54. Sasaki, N., Masaki, I., et al. Transcriptional Sequencing: A Method for DNA Sequencing Using RNA Polymerase. Proc. Natl. Acad. Sci. USA, 95:3455-3460,1998.
55. Sharp P. RNA interference-2001. Genes Dev 15:485-490, 2001.
56. Sonenberg N. mRNA translation: influence of the 5' and 3' untranslated regions. Curr Opin Gen Dev 4:310-315, 1994.
57. Stark B, Kole R, Bowman E, Altman S. Ribonuclease P: an enzyme with an essential RNA component. Proc Natl Acad Sci USA 75:3717-3721, 1978.
58. Szymański M, Barciszewski. Beyond the proteome: non-coding regulatory RNAs. Gen Biol 3:R0005.1-0005.8, 2002.
59. Tang T, Bachellerie J-P, Rozhdestvensky Y, Bortolin M, Huber H, Drungoski M. Elge T, Brosius J, Hüttenhofer A. Identification of 86 candidates for small non-messenger RNAs from archaeon Archaeoglobus fulgidus. Proc Natl Acad Sci USA 99:7536-7541, 2002.
60. Tycowski K, Smith C, Shu M, Steitz J. A small nucleolar RNA requirement for the site-specific ribose methylation of rRNA in Xenopus. Proc Natl Acad Sci USA 93:14480-14485, 1996.
61. Vance V, Vaucheret H. RNA silencing in plants-defense and counterdefense. Science 292:2277-2280, 2001.
62. Van der Velden A, Thomas A. The role of the 5' untranslated region of an mRNA in translation regulation during development. Int J Biochem Cell Biol 31:87-106, 1999.
63. Veenstra G.J.C., et al. 1999. Translation of Maternal TATA-Binding Protein mRNA Potentiates Basal but Not Activated Transcription in Xenopus Embryos at the Midblastula Transition. Mol. Cell. Biol., p.7972-7982.
64. Venter J, Adams MD, Myers EW, Li PW, Mural RJ, Sutton GG, Smith HO, Yandell M, Evans CA, Holt RA, Gocayne JD, et al. The sequence of the human genome. Science 291:1304-1351,2001.
65. Walter P, Blobel G. Signal recognition particle contains a 7S RNA essential for protein translocation across the endoplasmic reticulum. Nature 299:691-698, 1982.
66. Wassarmann K, Repoila F, Rosenow C, Storz G, Gottesman S. Identification of novel small RNAs using comparative genomics and microarrays. Genes Dev 15:1637-1651,2001.
67. Weinstein L, Steitz J. Guided tours: from precursor snoRNA to functional snoRNP. Curr Opin Cell Biol 11:376-384, 1999.
68. Yu Y, Scharl E, Smith C, Steitz J. In: The RNA world, 2nd edition (eds. Gesteland R, Cech T, Atkins J, pp.487-524, Cold Spring Harbor Laboratory Press, New York, 1999.

## Claims

1. A method for direct identification of RNA transcripts generated from a DNA sample obtained from a eukaryotic or prokaryotic cell population, wherein the DNA sample comprises a genomic library or a portion of a genomic library, comprising the steps of:
a) preparing DNA fragments from the DNA sample such that each fragment has only one RNA polymerase promoter and wherein each RNA polymerase promoter is endogenous to the genome of the cell population;
b) providing a wild type RNA polymerase, wherein the RNA polymerase is endogenous to the cell population;
c) transcribing the DNA, fragments with the wild type RNA polymerase in the presence of ribonucleoside triphosphates (rNTPs) and transcription terminating nucleotides to obtain transcription termination products;
d) separating the transcription termination products; and
e) determining the sequence of the RNA transcripts from the separated transcription termination products.

2. The method of claim 1, wherein the wild type RNA polymerase is provided in a transcriptionally active cell extract.

3. The method of claim 1, wherein the RNA polymerase is E. coli RNA polymerase.

4. The method of claim 1, wherein the rNTPs and transcription terminating nucleotides are present in an equal molar ratio.

5. A method for detecting differences in the sequence of RNA transcripts generated from DNA samples prepared from two different cell populations comprising the steps of:
a) providing a first DNA sample from a first population of prokaryotic or eukaryotic cells and a second DNA sample from a second population of prokaryotic or eukaryotic cells;
b) preparing DNA fragments from the first and second DNA samples to obtain a first and second set of DNA fragments such that each fragment of the first set of fragments has only one RNA polymerase promoter, wherein each RNA polymerase promoter is endogenous to the genome of the first cell population; and each fragment of the second set of fragments has only one RNA polymerase promoter, wherein each RNA polymerase promoter is endogenous to the genome of the second cell population;
c) transcribing the first and second set of DNA fragments using a wild type RNA polymerase, to obtain a first and second set of RNA transcripts;
d) determining the sequence of the first and second set of RNA transcripts; and
e) comparing the sequence of the first and second set of RNA transcripts to detect differences between the first and second set of RNA transcripts.

6. The method of claim 5, wherein the wild type RNA polymerase is provided in a transcriptionally active cell extract.

7. The method of claim 5, wherein the RNA polymerase is E. coli RNA polymerase.

8. The method of claim 5, wherein the step of determining the sequence of the first and second set of RNA transcripts is performed by transcribing the first and second sets of DNA fragments with the wild type RNA polymerase in the presence of ribonucleoside triphosphates (rNTPs) and transcription terminating nucleotides to obtain transcription termination products; separating the transcription termination products by gel electrophoresis or high pressure liquid chromatography; and determining the sequence of the RNA transcripts from the separated transcription termination products.

9. The method of claim 8, wherein the 5'-rNTPs and transcription terminating nucleotides are present in an equal molar ratio.

10. The method of claim 5, wherein the first and second cell populations are obtained from the same organism.

11. The method of claim 5, wherein the first cell population is from a tumor and the second cell population is from a tissue other than the tumor.

## Patentansprüche

1. Verfahren zur direkten Identifizierung von RNA-Transkripten, erzeugt von einer DNA-Probe, welche von einer eukaryotischen oder prokaryotischen Zellpopulation erhalten wurde, wobei die DNA-Probe eine Genom-Bibliothek oder einen Teil einer Genom-Bibliothek umfasst, umfassend die Schritte von:
a) Herstellen von DNA-Fragmenten von der DNA-Probe, so dass jedes Fragment nur einen RNA-Polymerasepromotor aufweist, und wobei jeder RNA-Polymerasepromotor zum Genom der Zellpopulation endogen ist;
b) Bereitstellen einer Wildtyp-RNA-Polymerase, wobei die RNA-Polymerase zu der Zellpopulation endogen ist;
c) Transkribieren der DNA-Fragmente mit der Wildtyp-RNA-Polymerase in der Gegenwart von Ribonukleosidtriphosphaten (rNTPs) und Transkriptions-terminierenden Nukleotiden, um Transkriptionsterminationsprodukte zu erhalten;
d) Trennen der Transkriptionsterminationsprodukte; und
e) Bestimmen der Sequenz der RNA-Transkripte von den getrennten Transkriptionsterminationsprodukten.

2. Verfahren nach Anspruch 1, wobei die Wildtyp-RNA-Polymerase in einem transkriptionell aktiven Zellextrakt bereitgestellt wird.

3. Verfahren nach Anspruch 1, wobei die RNA-Polymerase E. coli-RNA-Polymerase ist.

4. Verfahren nach Anspruch 1, wobei die rNTPs und Transkriptions-terminierenden Nukleotide in einem äquimolaren Verhältnis vorhanden sind.

5. Verfahren zum Nachweisen von Unterschieden in der Sequenz von RNA-Transkripten, erzeugt von DNA-Proben, welche von zwei unterschiedlichen Zellpopulationen hergestellt wurden, umfassend die Schritte von:
a) Bereitstellen einer ersten DNA-Probe von einer ersten Population von prokaryotischen oder eukaroytischen Zellen und einer zweiten DNA-Probe von einer zweiten Population von prokaryotischen oder eukaryotischen Zellen;
b) Herstellen von DNA-Fragmenten von den ersten und zweiten DNA-Proben, um einen ersten und zweiten Satz von DNA-Fragmenten zu erhalten, so dass jedes Fragment von dem ersten Satz von Fragmenten nur einen RNA-Polymerasepromotor aufweist, wobei jeder RNA-Polymerasepromotor zum Genom der ersten Zellpopulation endogen ist; und jedes Fragment von dem zweiten Satz von Fragmenten nur einen RNA-Polymerasepromotor aufweist, wobei jeder RNA-Polymerasepromotor zum Genom der zweiten Zellpopulation endogen ist;
c) Transkribieren des ersten und zweiten Satzes von DNA-Fragmenten unter Verwendung einer Wildtyp-RNA-Polymerase, um einen ersten und zweiten Satz von RNA-Transkripten zu erhalten;
d) Bestimmen der Sequenz des ersten und zweiten Satzes von RNA-Transkripten; und
e) Vergleichen der Sequenz des ersten und zweiten Satzes von RNA-Transkripten, um Unterschiede zwischen dem ersten und zweiten Satz von RNA-Transkripten nachzuweisen.

6. Verfahren nach Anspruch 5, wobei die Wildtyp-RNA-Polymerase in einem transkriptionell aktiven Zellextrakt bereitgestellt wird.

7. Verfahren nach Anspruch 5, wobei die RNA-Polymerase E. coli-RNA-Polymerase ist.

8. Verfahren nach Anspruch 5, wobei der Schritt des Bestimmens der Sequenz des ersten und zweiten Satzes von RNA-Transkripten durchgeführt wird durch Transkribieren der ersten und zweiten Sätze von DNA-Fragmenten mit der Wildtyp-RNA-Polymerase in der Gegenwart von Ribonukleosidtriphosphaten (rNTPs) und Transkriptions-terminierenden Nukleotiden, um Transkriptionsterminationsprodukte zu erhalten; Trennen der Transkriptionsterminationsprodukte durch Gelelektrophorese oder Hochdruckflüssigkeitschromatographie; und Bestimmen der Sequenz der RNA-Transkripte von den getrennten Transkriptionsterminationsprodukten.

9. Verfahren nach Anspruch 8, wobei die 5'-rNTPs und Transkriptions-terminierenden Nukleotide in einem äquimolaren Verhältnis vorhanden sind.

10. Verfahren nach Anspruch 5, wobei die ersten und zweiten Zellpopulationen aus dem selben Organismus erhalten werden.

11. Verfahren nach Anspruch 5, wobei die erste Zellpopulation von einem Tumor stammt und die zweite Zellpopulation von einem anderen Gewebe als dem Tumor stammt.

## Revendications

1. Procédé pour l'identification directe de transcrits d'ARN généré à partir d'un échantillon d'ADN obtenu à partir d'une population de cellules eucaryotes ou procaryotes, dans lequel l'échantillon d'ADN comprend une banque génomique ou une portion d'une banque génomique, comprenant les étapes consistant à :
a) préparer des fragments d'ADN à partir de l'échantillon d'ADN, de sorte que chaque fragment ait seulement un promoteur de polymérase d'ARN et dans lequel chaque promoteur de polymérase d'ARN est endogène au génome de la population cellulaire ;
b) fournir une polymérase d'ARN de type sauvage, dans laquelle la polymérase d'ARN est endogène à la population cellulaire ;
c) transcrire les fragments d'ADN avec la polymérase d'ARN de type sauvage, en présence de ribonucléoside triphosphates (rNTP) et de nucléotides à terminaison de transcription pour obtenir des produits de terminaison de transcription ;
d) séparer les produits de terminaison de transcription et
e) déterminer la séquence des transcrits d'ARN des produits de terminaison de transcription séparés.

2. Procédé selon la revendication 1, dans lequel la polymérase d'ARN de type sauvage est fournie dans un extrait de cellule active de manière transcriptionnelle.

3. Procédé selon la revendication 1, dans lequel la polymérase d'ARN est la polymérase d'ARN d'E.coli.

4. Procédé selon la revendication 1, dans lequel les rNTP et les nucléotides à terminaison de transcription sont présents dans un rapport molaire égal.

5. Procédé de détection des différences dans la séquence de transcrits d'ARN générés à partir des échantillons d'ADN préparés à partir de deux populations de cellules différentes comprenant les étapes consistant à :
a) fournir un premier échantillon d'ADN provenant d'une première population de cellules procaryotes ou eucaryotes, et un second échantillon d'ADN à partir d'une seconde population de cellules procaryotes ou eucaryotes ;
b) préparer des fragments d'ADN provenant du premier et du second échantillons d'ADN pour obtenir un premier et un second ensembles de fragments d'ADN, de sorte que chaque fragment du premier ensemble de fragments ait seulement un promoteur de polymérase d'ARN, dans lequel chaque promoteur de polymérase d'ARN est endogène au génome de la première population cellulaire, et chaque fragment du second ensemble de fragments a seulement un promoteur de polymérase d'ARN, dans lequel chaque promoteur de polymérase d'ARN est endogène au génome de la seconde population cellulaire ;
c) transcrire le premier et le second ensemble de fragments d'ADN en utilisant une polymérase d'ARN de type sauvage pour obtenir un premier et un second ensembles de transcrits d'ARN ;
d) déterminer la séquence du premier et du second ensemble de transcrits d'ARN ; et
e) comparer la séquence du premier et du second ensembles de transcrits d'ARN pour détecter les différences entre le premier et le second ensembles de transcrits d'ARN.

6. Procédé selon la revendication 5, dans lequel la polymérase d'ARN de type sauvage est fournie dans un extrait de cellule actif d'un point de vue transcriptionnel.

7. Procédé selon la revendication 5, dans lequel la polymérase d'ARN est une polymérase d'ARN d'E.coli.

8. Procédé selon la revendication 5, dans lequel l'étape de détermination de la séquence du premier et du second ensembles de transcrits d'ARN est réalisée en transcrivant le premier et le second ensembles de fragments d'ADN avec la polymérase d'ARN de type sauvage en présence de triphosphates de ribonucléoside (rNTP) et de nucléotides à terminaison de transcription pour obtenir des produits de terminaison de transcription ; en séparant les produits de terminaison de transcription par électrophorèse sur gel ou chromatographie liquide haute pression ; et en déterminant la séquence des transcrits d'ARN des produits de terminaison de transcription séparés.

9. Procédé selon la revendication 8, dans lequel les 5'-rNTP et les nucléotides de terminaison de transcription sont présents selon un rapport molaire égal.

10. Procédé selon la revendication 5, dans lequel la première et la seconde populations cellulaires sont obtenues à partir du même organisme.

11. Procédé selon la revendication 5, dans lequel la première population cellulaire provient d'une tumeur et la seconde population cellulaire provient d'un tissu autre que la tumeur.
